# EUROPÄISCHE PATENTSCHRIFT

(11) **EP 3 693 355 B1**
(45) Veröffentlichungstag und Bekanntmachung des Hinweises auf die Patenterteilung: **10.11.2021**
(21) Anmeldenummer: 19155897.2
(22) Anmeldetag: 07.02.2019
(51) Int. Cl.: C07C 29/141, C07C 41/06, C07C 2/10, C07C 45/50, C07C 5/333, C07C 5/48, C07C 7/163, C07C 11/08, C07C 9/12, C07C 11/09, C07C 11/02, C07C 47/02, C07C 31/125, C07C 43/04, C07C 5/03, C07C 7/04

(54) **FLEXIBLE HERSTELLUNG VON MTBE ODER ETBE UND ISONONANOL**
FLEXIBLE MANUFACTURE OF MTBE OR ETBE AND ISONONANOL
PRODUCTION FLEXIBLE DE MTBE OU ETBE ET ISONONANOL

(43) Veröffentlichungstag der Anmeldung: 12.08.2020
(73) Patentinhaber: Evonik Operations GmbH, 45128 Essen (DE)
(72) Erfinder: STOCHNIOL, Guido, 45721 Haltern am See (DE); SCHALLENBERG, Jörg, 46286 Dorsten (DE); WINTERBERG, Markus, 45731 Waltrop (DE)
(74) Vertreter: Evonik Patent Association

(56) Entgegenhaltungen:
- EP-A1- 0 820 974
- EP-A1- 3 293 171
- DE-A1-102008 007 081

## Beschreibung

Die vorliegende Erfindung betrifft ein Verfahren zur flexiblen Herstellung von MTBE oder ETBE und Isononanol, bei der die Isobutan- und optional auch Butan-Anteile von technischen C4-Strömen flexibel zur Herstellung von MTBE oder ETBE und optional Isononanol genutzt werden können.

Die Herstellung von MTBE (Methyl-tert-butylether) aus olefinhaltigen C4-Strömen erfolgt durch Veretherung des im C4-Strom vorhandenen Isobutens mit Methanol. ETBE erfolgt durch Veretherung mit Ethanol. Verfahren zur Herstellung von MTBE oder ETBE sind im Stand der Technik bekannt und werden beispielsweise eingesetzt um Isobutene aus C4-Strömen zu entfernen, da dies aufgrund eines zu n-Butenen sehr ähnlichen Siedepunktes über destillative Trennverfahren schwierig zu realisieren ist.

Isononanol wird üblicherweise durch eine Oligomerisierung, bei dem die Butene des eingesetzten C4-Stroms unter Verwendung eines Katalysators zu C8-Olefinen umgesetzt werden, eine Hydroformylierung der C8-Olefine mit Synthesegas (Mischung aus CO und H₂) zu den entsprechenden C9-Aldehyden, insbesondere Isononanal, und nachfolgende Hydrierung des Aldehyds Isononanal zum Alkohol Isononanol erzeugt. Sowohl Verfahren zur Oligomerisierung von Butenen aus C4-Strömen zu C8-Olefinen, als auch Verfahren zu Hydroformylierung von C8-Olefinden sind hinlänglich bekannt und im Stand der Technik beschrieben.

Die Quelle für die genannten Verfahren sind in der Regel Steamcracker, in denen aus Naphtha kurzkettige Olefine wie Ethylen oder Propylen aber auch eine Butadien-, Isobuten- und n-Butenhaltige C4-Fraktion (sogenanntes Crack-C4) gewonnen werden kann, die beispielsweise durch eine MTBE- oder ETBE-Synthese vom Isobuten befreit werden muss, um einer nachgeschalteten Oligomerisierung zugeführt werden zu können.

Als Rohstoffquelle in der petrochemischen Industrie stehen grundsätzlich auch andere C4-Ströme zur Verfügung. In eher kraftstofforientierten Prozessen wird typischerweise eine C4-Fraktion aus katalytischen Crackverfahren (Fluid Catalytic Cracking / FCC) eingesetzt. Diese FCC-C4-Fraktion unterscheidet sich in der Zusammensetzung jedoch sehr deutlich von den genannten Crack-C4-Fraktionen aus Steamcrackern. FCC-C4-Ströme enthalten häufig hohe Anteile an Isobutan (im Bereich von 20 bis 30 Gew.-%), welches in der chemischen Industrie kaum Anwendung findet. Gleiches gilt für Feldbutane, womit die von Erdgasen oder Erdölbegleitgasen abgetrennte C4-Fraktion gemeint ist, die ebenfalls hohe Anteile an Isobutan aufweist.

Die Anteile von inerten Alkanen wie Isobutan oder n-Butan in C4-Kohlenwasserstoffströmen müssen irgendwann abgetrennt werden, damit sie sich in den chemischen Prozessen nicht anreichern. Das Problem ist, dass die abgetrennten Alkane inert sind und in chemischen Prozessen nicht eingesetzt werden können. Stattdessen werden Alkane beispielsweise nur als Brenngas oder Treibgas verwendet oder einfach verbrannt.

Die Aufgabe der vorliegenden Erfindung bestand darin diese Alkane wirtschaftlich nutzbar zu machen, um sie in variablen Mengen in (petro)chemischen Verfahren einsetzen zu können. Außerdem sollte das Verfahren möglichst flexibel ausgestaltet werden, um auf variierende Bedürfnisse der erzeugten Produkte, beispielsweise den Bedarf an MTBE oder ETBE für Kraftstoffe oder die Nutzung von reinem Isobuten aus einer optionalen MTBE-Spaltung, reagieren zu können.

Die Aufgabe konnte durch das Verfahren nach Anspruch 1 gelöst werden. Bevorzugte Ausführungsformen sind in den Unteransprüchen angegeben.

Gegenstand der vorliegenden Erfindung ist ein Verfahren zur flexiblen Herstellung von MTBE und Isononanol, welches die folgenden Schritte umfasst:
a) Bereitstellen eines C4-Kohlenwasserstoffstroms, welcher zumindest n-Butene (1-Buten und 2-Buten), n-Butan und Isobutan enthält, als ersten Einsatzstrom und destillative Abtrennung zumindest eines Teils des Isobutans und zumindest eines Teils des 1-Butens aus dem ersten Einsatzstrom, wobei der C4-Kohlenwasserstoffstrom ein Raffinat-II-Strom ist;
b) Verwendung des aus dem ersten Einsatzstrom abgetrennten Isobutans und zusätzliches Isobutan, welches aus einem Feldbutanstrom oder einem FCC-C4-Strom abgetrennt worden ist, als zweiten Einsatzstrom;
c) Zuführen des ersten Einsatzstroms zu einer Oligomerisierung, in der die n-Butene katalytisch zu C8-Olefinen oligomerisiert werden;
d) Zuführen der in der Oligomerisierung in Schritt c) gebildeten C8-Olefine zu einer Hydroformylierung, in der C8-Olefine mit Synthesegas katalytisch zu C9-Aldehyden umgesetzt werden;
e) Zuführen der C9-Aldehyde aus der Hydroformylierung in Schritt d) zu einer Hydrierung, bei der C9-Aldehyde katalytisch zu Isonanol umgesetzt werden;
f) Zuführen des zweiten Einsatzstroms zu einer Dehydrierung, wodurch ein Dehydriergemisch erhalten wird, welches zumindest Isobuten und nicht umgesetztes Isobutan enthält und optional Abtrennen des Isobutans vom Dehydriergemisch;
g) Zuführen des Isobutens aus Schritt f) zu einer MTBE- oder ETBE-Synthese, in der Isobuten mit Methanol katalytisch zu MTBE oder mit Ethanol katalytisch zu ETBE umgesetzt wird, oder zu einer Isobutendimerisierung, bei der Isobuten katalytisch dimerisiert wird.
   Der Anspruchsformulierung sollte zu entnehmen sein, dass nicht alle Schritte chronologisch in der gelisteten Abfolge durchgeführt werden müssen, sondern dies nur zur besseren Verständlichkeit in den Schritten a) bis g) formuliert worden ist. Nur die Bildung von Isononanol (Schritte a) und c) bis e)) und die Bildung von MTBE oder ETBE bzw. Isobutendimeren (Schritte b), f) und g)) sollten jeweils chronologisch in der angegebenen Reihenfolge durchgeführt werden. Die Bildung von Isononanol, also die Schritte a) und c) bis e), und die Bildung von MTBE oder ETBE bzw. Isobutendimeren, also die Schritte b), f) und g), können somit grundsätzlich gleichzeitig erfolgen.

Mit dem erfindungsgemäßen Verfahren können Isobutananteile aus C4-Kohlenwasserstoffströmen, vorzugsweise von Raffinat-II-Strömen, und Isobutananteile aus FCC-C4-Strömen und Feldbutanen durch Dehydrierung nutzbar gemacht werden, beispielsweise für die Herstellung von MTBE oder für die Dimerisierung. Durch das erfindungsgemäße Verfahren kann auch flexibel auf Marktbedürfnisse reagiert werden, insofern als dass der Anteil des aus dem C4-Kohlenstoffstroms abgetrennten Isobutans verringert oder erhöht wird, um mehr oder weniger MTBE oder Isobutendimere zu bilden.

Im ersten Schritt a) wird ein C4-Kohlenwasserstoffstrom, welcher zumindest n-Butene (1-Buten und 2-Buten), n-Butan und Isobutan enthält, als erster Einsatzstrom bereitgestellt. Im Sinne der vorliegenden Erfindung ist unter dem Begriff C4-Kohlenwasserstoffstrom ein C4-Olefin enthaltender Kohlenwasserstoffstrom zu verstehen. Technisch verfügbare C4-Kohlenwasserstoffströme enthalten neben den Butenen zumeist auch Anteile an den entsprechenden Alkanen n-Butan und Isobutan. Der C4-Kohlenwasserstoffstrom ist ein Raffinat-II-Strom, insbesondere aus einer Crack-C4-Fraktion eines Steamcrackers oder aus einer C4-Fraktion (FCC-C4) eines Fluid Catalytic Cracker.

Von diesem C4-Kohlenwasserstoffstrom als erstem Einsatzstrom wird in Schritt a) zumindest ein Teil des im Strom vorhandenen Isobutans und zumindest ein Teil des 1-Butens destillativ abgetrennt. Die Mischung aus Isobutan und 1-Buten wird als Leichtsiederfraktion am Kopf der Destillationskolonne abgenommen. Die Destillationsbedingungen, insbesondere Temperatur und Druck, sind dem Fachmann beispielsweise aus der DE 10 2008 007 081 A1 bekannt. Die Leichtsiederfraktion, also die Mischung aus Isobutan und 1-Buten kann dann weiter destillativ aufgearbeitet werden, um Isobutan und 1-Buten voneinander zu trennen. Entsprechende Angaben zur Destillation dieser Leichtsiederfraktion aus Isobutan und 1-Buten können ebenfalls der DE 10 2008 007 081 A1 entnommen werden.

Das wie vorgenannt abgetrennte reine 1-Buten kann flexibel verwertet werden. Einerseits kann es in variabler Menge zum C4-Kohlenwasserstoffstrom nach Schritt a) (also nach Abtrennung von Isobutan und 1-Buten) zugegeben werden. Dadurch kann die 1-Buten-Konzentration im C4-Kohlenwasserstoffstrom so eingestellt werden, dass die nachfolgende Oligomerisierung zu gewünschten weniger verzweigten Oligomeren führt. Andererseits kann das 1-Buten zumindest teilweise aus dem Verfahren ausgeschleust und verkauft oder in eigenen Anlagen als Edukt für andere chemische Verfahren verwendet werden.

Das aus dem ersten Einsatzstrom abgetrennte Isobutan wird in Schritt b) zusammen mit weiterem Isobutan, welches aus einem Feldbutanstrom oder einem FCC-C4-Strom abgetrennt worden ist, als zweiter Einsatzstrom zur Herstellung von MTBE bzw. Isobutendimeren verwendet. Die Menge des aus dem ersten Einsatzstrom abgetrennten Isobutans kann variiert werden, um die Menge des zu bildenden MTBE bzw. der zu bildenden Isobutendimere dem Marktbedarf anzupassen.

Der erste Einsatzstrom wird gemäß den Schritten c) bis e) zur Herstellung von Isononanol eingesetzt. Dazu wird der erste Einsatzstrom zunächst in Schritt c) zu einer Oligomerisierung geführt, in der die im Strom befindlichen n-Butene katalytisch zu C8-Olefinen oligomerisiert werden. Die Oligomerisierung erfolgt üblicherweise in mindestens einem geeigneten Reaktor. Es sind natürlich auch Ausführungsformen denkbar, bei der mehrere gleichzeitig betriebene Reaktoren parallel geschaltet oder in Reihe geschaltet vorliegen. Als Reaktor können alle dem Fachmann bekannten Reaktoren eingesetzt werden, die für die Oligomerisierung geeignet sind, bspw. Rohrreaktoren, Rohrbündelreaktoren, Riser-Settler-Reaktoren, Slurry-Reaktoren.

Der Reaktor enthält einen Oligomerisierungskatalysator zur Durchführung der Oligomerisierung, insbesondere einen heterogenen Oligomerisierunsgkatalysator. Der Oligomerisierungskatalysator liegt dabei insbesondere in Form eines Granulats, eines Extrudats oder in Tablettenform vor.

Der (heterogene) Oligomerisierungskatalysator ist vorzugsweise ein Übergangsmetall-haltiger Oligomerisierungskatalysator. Das Übergangsmetall bzw. die entsprechend eingesetzte Übergangsmetallverbindung ist dabei vorzugsweise auf einem Trägermaterial, besonders bevorzugt einem auf Alumosilicat basierenden Trägermaterial, angeordnet. Neben dem Trägermaterial können zusätzlich Binder auf Basis von Aluminiumoxid (Al₂O₃) oder Siliciumoxid (SiO₂) im Oligomerisierungskatalysator eingesetzt werden. Als Übergangsmetallverbindungen für die erfindungsgemäße eingesetzten Oligomerisierungskatalysatoren eignen sich insbesondere Verbindungen von Nickel, Cobalt, Chrom, Titan und Tantal. Bevorzugt sind Nickel- und Cobalt-Verbindungen, besonders bevorzugt sind Nickel-Verbindungen. In einer bevorzugten Ausführungsform umfasst der erfindungsgemäße Oligomerisierungskatalysator eine Nickelverbindung, vorzugsweise Nickeloxid, auf einem Alumosilicat-Trägermaterial.

Die Oligomerisierung kann bei einer Temperatur im Bereich von 50 bis 200 °C, vorzugsweise 60 bis 180 °C, bevorzugt im Bereich von 60 bis 130°C durchgeführt werden. Der Druck kann von 10 bis 70 bar, bevorzugt von 20 bis 55 bar betragen.

Nach der Oligomerisierung sollte das erhaltene Oligomerisat dadurch aufgearbeitet werden, dass die gebildeten C8-Olefine und möglicherweise gebildete höheren Oligomere (C16, C20, etc.) vom restlichen Gemisch (auch: Rohbutan), welches zumindest n-Butan und nicht umgesetzte Butene enthält, abgetrennt wird. Das kann beispielsweise in bekannter Weise mittels Destillation gemäß DE 10 2008 007 081 A1 erfolgen. Die abgetrennten C8-Olefine werden im nächsten Schritt einer Hydroformylierung zugeführt.

Von dem restlichen Gemisch, von dem die C8-Olefine und die möglicherweise gebildeten höheren Oligomere abgetrennt worden sind, können die Butene abgetrennt werden, mittels Extraktion entsprechend DE 10 2008 007 081 A1. Die Butene aus dem restlichen Gemisch können nach Abtrennung eines Großteils der n-Butane zur Oligomerisierung zurückgeführt werden. Das restliche Gemisch, insbesondere die enthaltenen Butane, werden zumindest teilweise aus dem Teil des Verfahrens für den ersten Einsatzstrom ausgeschleust, um eine Anreicherung von inerten Butanen, insbesondere in der Oligomerisierung, zu verhindern.

In einer bevorzugten Ausführungsform werden die Butene jedoch nicht abgetrennt, sondern das gesamte restliche Gemisch einer Hydrierung zugeführt, um die restlichen Butene zu Butanen zu hydrieren (siehe DE 10 2008 007 081 A1). Das erhaltene Butan kann nach optionaler weiterer Aufarbeitung als Brenn- oder Treibgas eingesetzt oder - wie nachfolgend erläutert - zusammen mit dem in Schritt a) abgetrennten Isobutan als zweites Einsatzgemisch verwendet werden.

Die bei der Oligomerisierung gebildeten C8-Olefine werden dann in Schritt d) einer Hydroformylierung zugeführt, bei der C8-Olefine mit Synthesegas katalytisch zu C9-Aldehyden umgesetzt werden. Synthesegas bei der Hydroformylierung betrifft bekanntermaßen eine Mischung aus CO und H₂.

Die Hydroformylierung ist vorzugsweise eine homogen katalysierte Hydroformylierung, bei der das Katalysatorsystem (vollständig) gelöst in der flüssigen Phase des Reaktionsgemisches vorliegt. Das Katalysatorsystem der ersten Hydroformylierung umfasst vorzugsweise ein Übergangsmetall aus der 8. oder 9. Gruppe des Periodensystems der Elemente (PSE) und mindestens einen organischen Phosphor-haltigen Liganden.

Als Übergangsmetall können insbesondere Eisen, Ruthenium, Iridium, Cobalt oder Rhodium, vorzugsweise Cobalt oder Rhodium eingesetzt werden. Als katalytische aktive Spezies werden üblicherweise (Liganden)-Carbonyl-Komplexe der Metallatome diskutiert, die sich unter den bei der Hydroformylierung vorherrschenden Drücken und Temperaturen im flüssigen Reaktionsgemisch bilden.

Die Hydroformylierung kann in Anwesenheit eines Lösemittels durchgeführt werden, wobei das Lösemittel mit dem Hydroformylierungsverfahren kompatibel sein sollte. Als Lösemittel können die dem Fachmann bekannten Lösemittel für die klassische Hydroformylierung verwendet werden, beispielsweise Alkane, aromatische Kohlenwasserstoffe, Wasser, Ether, Ester, Ketone, Alkohole und die Reaktions- oder Nebenprodukte der Hydroformylierung wie Aldehyde und Kondensationsprodukte der Aldehyde.

Die Hydroformylierung kann bei einem Druck von 10 bis 400 bar, vorzugsweise 15 bis 250 bar durchgeführt werden. Die Temperatur bei der Hydroformylierung kann 70 bis 250 °C, vorzugsweise 100 bis 200 °C betragen.

Die Hydroformylierung sollte ebenfalls in mindestens einem Reaktor durchgeführt werden. Es sind natürlich auch Ausführungsformen denkbar, bei der mehrere gleichzeitig betriebene Reaktoren parallel geschaltet oder in Reihe geschaltet vorliegen. Als Reaktor für die Hydroformylierung kommen alle dem Fachmann bekannten und für die Hydroformylierung geeigneten Reaktortypen, insbesondere Gas-Flüssig-Reaktoren, in Betracht. Geeignete Reaktortypen sind insbesondere Rührkesselreaktoren, Blasensäulenreaktoren, kaskadierte Blasensäulenreaktoren, Jetloopreaktoren oder Schlaufenreaktoren.

Die bei der Hydroformylierung in Schritt d) gebildeten C9-Aldehyde können danach in bekannter Weise aufgearbeitet (z. B. Abtrennung des Katalysators) und vom Reaktionsgemisch, beispielsweise mittels Extraktion oder Destillation in bekannter Weiseabgetrennt werden. Die erhaltenen C9-Aldehyde werden nachfolgend in Schritt e) einer Hydrierung zugeführt, bei der die C9-Aldehyde katalytisch unter Zugabe von Wasserstoff zu Isononanol umgesetzt werden.

Die Hydrierung in Schritt e) kann in der Gasphase oder in der Flüssigphase erfolgen, wobei die Hydrierung in der Flüssigphase bevorzugt ist. Der Gesamtdruck in Schritt e) beträgt vorzugsweise 5 bis 100 bar, vorzugsweise 15 bis 50 bar. Bei Gasphasenhydrierungen sind auch geringere Drücke denkbar. Die Hydrierung kann einstufig, also in einem Reaktor, oder mehrstufig, also in mehreren in Reihe geschalteten Reaktoren durchgeführt werden. Im letzteren Fall können die Drücke in den einzelnen Reaktoren gleich oder unterschiedlich voneinander sein, mit der Maßgabe. dass die Drücke in den Reaktoren jeweils in den oben genannten Bereichen liegen.

Die Hydrierung in Schritt e) kann bei Temperaturen von 120 bis 220 °C betragen, vorzugsweise140 bis 180 °C. Das betrifft sowohl die Gas- als auch die Flüssigphasenhydrierung. Als Katalysatoren können zur Hydrierung in Schritt c) Kupfer-, Nickel-, Kupfer/Nickel-, Kupfer/Chrom-, Kupfer/Chrom/Nickel-, Zink/Chrom-, Nickel/Molybdän-Katalysatoren eingesetzt werden. Die Katalysatoren können trägerfrei sein, auf Trägern, wie beispielsweise Aluminiumoxid, Titandioxid, Zirkondioxid oder Siliziumdioxid oder Mischungen davon aufgebracht sein.

Bevorzugte Katalysatoren, die bei der Hydrierung in Schritt e) eingesetzt werden, enthalten, jeweils von 0,3 bis 15 Gew.-% Kupfer und/oder Nickel sowie optional als Aktivatoren 0,05 bis 3,5 Gew.-% Chrom und/oder von 0,01 bis 1,6 Gew.-%, vorzugsweise 0,02 bis 1,2 Gew.--% einer Alkalikomponente, wie z. B. Natrium oder Kalium, auf einem Trägermaterial, vorzugsweise Aluminiumoxid und Siliziumdioxid oder Mischungen davon. Die Gewichtsprozentangaben beziehen sich jeweils auf die Gesamtzusammensetzung des Katalysators vor der Aktivierung mit Wasserstoff.

Die Katalysatoren können als Granulat, in Form von Pellets, Tabletten, Zylindern, Strangextrudaten oder Ringen eingesetzt werden. Sie werden zweckmäßig vor ihrem Einsatz aktiviert, z. B. durch Erhitzen im Wasserstoffstrom.

Der zweite Einsatzstrom, der das Isobutan aus dem C4-Kohlenwasserstoffstrom und dem Isobutan, welches aus einem Feldbutanstrom oder einem FCC-C4-Strom abgetrennt worden ist, wird gemäß Schritt f) zunächst zu einer Dehydrierung geführt, wodurch ein Dehydriergemisch erhalten wird, welches zumindest Isobuten und nicht umgesetztes Isobutan enthält.

Die Dehydrierung in Schritt f) ist eine endotherme Reaktion und kann bei Temperaturen zwischen 400 und 800 °C, vorzugsweise zwischen 500 und 650 °C durchgeführt werden. Die benötigte Wärmeenergie kann beispielsweise durch äußere Beheizung zugeführt werden oder in der Variante einer oxidativen Dehydrierung (Zugabe von Sauerstoff, um den freiwerdenden Wasserstoff aus dem System zu entfernen) zumindest partiell im System erzeugt werden. Die Dehydrierung in Schritt f) kann zur Verbesserung der Selektivität unter Verwendung eines Katalysators durchgeführt werden. Der in Schritt f) eingesetzte Katalysator enthält vorzugsweise eine metallische Komponente und kann insbesondere eine metallische Komponente basierend auf Chrom oder Platin auf einem Silica- oder Aluminaträgermaterial enthalten. Geeignete Reaktoren für die Dehydrierung sind Festbett-, Fließbett, Wirbelschicht- oder Radialstromreaktoren.

Die Dehydrierung ist eine reversible Reaktion (Hydrierung), d. h. der zu erreichende Umsatz wird von der Gleichgewichtslage bestimmt. Die Gleichgewichtslage kann durch Einstellung von Temperatur, Druck, Stoffmengenkonzentration und/oder über die Verweilzeit beeinflusst werden. Der Druck bei der Dehydrierung ist deshalb vorzugsweise relativ gering und beträgt 0,03 bis 8 bar, vorzugsweise 0,3 bis 6 bar.

Von dem erhaltenen Dehydriergemisch kann das nicht umgesetzte Isobutan abgetrennt, vorzugsweise mittels Destillation, werden. Wird das Isobutan nicht abgetrennt, sondern das Dehydriergemisch direkt zur MTBE- oder ETBE-Synthese oder der Isobutendimerisierung geführt, kann - nach Abtrennung des gebildeten MTBE - reines Isobutan erhalten werden, das danach genutzt werden kann. In einer bevorzugten Ausführungsform wird das abgetrennte Isobutan zurück zur Dehydrierung (Schritt f)) geführt.

Das gebildete Isobuten kann im nachfolgenden Schritt g) zu einer MTBE- oder ETBE-Synthese, in der Isobuten mit Methanol katalytisch zu MTBE oder mit Ethanol katalytisch zu ETBE umgesetzt wird, oder zu einer Isobutendimerisierung, bei der Isobuten katalytisch dimerisiert wird, geführt werden.

Die MTBE- oder ETBE-Synthese ist dem Fachmann grundsätzlich bekannt. Für die Herstellung von MTBE oder ETBE aus Isobutenhaltigen Strömen können insbesondere saure lonenaustauschharze (Sulfonsäuregruppen) als heterogene Katalysatoren eingesetzt. Die MTBE- oder ETBE-Synthese kann in einem oder mehreren in Reihe geschalteten Reaktoren stattfinden. Der Katalysator wird vorzugsweise als Festbettkatalysator eingesetzt. Da die Bildung von MTBE oder ETBE eine Gleichgewichtsreaktion darstellt, kann es zweckmäßig sein mindestens eine Reaktivdestillationskolonne einzusetzen, in der gleichzeitig die Reaktion und die Abtrennung des MTBE erfolgen. Bei der Reaktivdestillation sollte ein Druck im Bereich von 3 bis 15 bar und eine Temperatur in der Reaktionszone von 55 bis 75 °C vorliegen.

Das MTBE oder ETBE kann zum Beispiel in Kraftstsoffen als Klopfschutzmittel verwendet werden. Abhängig vom Bedarf kann das in Schritt g) hergestellte MTBE oder ETBE auch in einem nachfolgenden Schritt in dem Fachmann bekannter Weise gespalten werden, um reines Isobuten zu bilden. Das reine Isobuten kann für unterschiedlichste Folgerprozessierung eingesetzt werden, beispielsweise die Dimerisierung, die Trimerisierung mit anschließender Folgechemie bspw. Oxierung, die Polymerisierung zum Polyisobuten oder die Herstellung von Methacrylsäure. Diese Folgeprozesse bzw. die daraus erhaltenen Produkten könnten auch aufgrund der aktuellen politischen Entscheidungen zukünftig eine (noch) größere Bedeutung erlangen. Das erfindungsgemäße Verfahren kann flexibel auf die sich möglicherweise ändernden Bedürfnisse angepasst werden.

Die Isobutendimerisierung ist dem Fachmann ebenfalls geläufig. Typische Verfahrensbedingungen für die Herstellung von Diisobuten sind beispielsweise der WO 02/064531 A1 zu entnehmen.

In einer anderen Ausführungsform des vorliegenden Verfahrens kann das zwischen der Oligomerisierung in Schritt c) und der Hydroformylierung in Schritt d) abgetrennte restliche Gemisch nach Vollhydrierung zum Butan zusammen mit dem Isobutan, welches in Schritt a) aus dem ersten Einsatzstrom abgetrennt worden ist, und dem Isobutan aus dem Feldbutan- oder FCC-C4-Strom als zweiter Einsatzstrom eingesetzt und zur Dehydrierung (Schritt f)) gefahren werden. Dadurch entstehen neben Isobuten auch n-Butene, die oligomerisiert werden können. Dadurch wird im Verfahrensstrang für den zweiten Einsatzstrom neben der Bildung von MTBE aus Isobuten auch eine Bildung von Isononanol aus den in der Dehydrierung gebildeten n-Butenen durch ein Verfahren entsprechend den Schritten c), d) und e) ermöglicht.

In dieser Ausgestaltung des Verfahrens wird in der Dehydrierung in Schritt f) nämlich nicht nur Isobuten gebildet, sondern auch n-Butene (1-Buten und 2-Buten), welche nach Abtrennung des Isobutens für die Oligomerisierung zur Verfügung stehen. Schritt f) dieser Ausführungsform des erfindungsgemäßen Verfahrens ersetzt demnach Schritt f) des vorher beschriebenen Verfahrens und zeichnet sich dadurch aus, dass der zweite Einsatzstrom zu einer Dehydrierung, die in dem Fachmann bekannter Weise bzw. wie oben beschrieben durchgeführt werden kann, geführt wird, wodurch ein Dehydriergemisch erhalten wird, welches zumindest n-Butene (1-Buten und 2-Buten) Isobuten, Butadien, nicht umgesetztes Butan und nicht umgesetztes Isobutan enthält. Von dem Dehydriergemisch kann das nicht umgesetzte Isobutan abgetrennt und zur Dehydrierung zurückgeführt werden. Die nicht umgesetzten Butane können nach der Oligomerisierung in Schritt i) abgetrennt und zur Dehydrierung zurückgeführt und/oder aus dem Verfahren ausgeschleust werden.

Das so erhaltene Dehydriergemisch enthält typischerweise Butadien, welches als Nebenprodukt bei der Dehydrierung von n-Butan entsteht. Butadien sollte jedoch vor der Oligomerisierung abgetrennt werden. Daher wird das Dehydriergemisch in Schritt g) zunächst einer selektiven Hydrierung zugeführt, in der Butadien unter Erhalt eines Hydriergemisches in Anwesenheit von Wasserstoff selektiv zu Buten hydriert wird.

Die Selektivhydrierung des Dehydriergemisches in Schritt g) findet üblicherweise in Anwesenheit von Wasserstoff und optional Kohlenmonoxid statt. Das molare Verhältnis von Wasserstoff zu Butadien liegt dabei insbesondere im Bereich von 2 : 1 und 1 : 1 (Wasserstoff: Butadien), vorzugsweise im Bereich von 1,5 : 1 und 1 : 1, besonders bevorzugt im Bereich von 1,2 : 1 und 1 : 1. Der Wasserstoff kann dabei separat zum Dehydriergemisch eindosiert werden oder dem Deyhdriergemisch vorher hinzugefügt werden.

Dem Dehydriergemisch, welches zur Selektivhydrierung geführt wird, kann zusätzlich Kohlenmonoxid zugegeben werden. Der Gehalt an Kohlenmonoxid im Dehydriergemisch beträgt vorzugsweise zwischen 0,05 und 20 ppm Kohlenmonoxid, besonders bevorzugt zwischen 0,5 und 5 ppm, jeweils bezogen auf die Gesamtmenge des Dehydriergemisches. Das Kohlenmonoxid kann dabei separat zum Dehydriergemisch eindosiert werden oder dem Dehydriergemisch vorher hinzugefügt werden.

Als Katalysator für die selektive Hydrierung in Schritt g) können heterogene Katalysatoren eingesetzt werden, die ein oder mehrere Metalle der 10. Gruppe des Periodensystems der Elemente enthalten, vorzugsweise Palladium und/oder Platin. Das Metall kann aufgrund von Kalzination partiell in Form seines Oxids vorliegen. Vorzugsweise liegt das Palladium und/oder das Platin auf einem Trägermaterial vor. Das Trägermaterial ist ausgewählt aus der Gruppe, bestehend aus Aluminiumoxid, Kieselgel und Aktivkohle, wobei bevorzugt Aluminiumoxid als Trägermaterial eingesetzt wird. Die Konzentration des Metalls im ersten Katalysator kann 0,01 bis 3 Gew.-%, vorzugsweise 0,1 bis 1 Gew.-%, besonders bevorzugt 0,3 bis 0,5 Gew.-%, jeweils bezogen auf die Gesamtmenge des Katalysators, betragen.

Die Eintrittstemperatur des Dehydriergemisches in die Selektivhydrierung liegt üblicherweise im Bereich von 0 bis 100 °C, vorzugsweise im Bereich von 20 bis 80 °C, besonders bevorzugt im Bereich von 30 bis 60 °C. Der Druck liegt üblicherweise im Bereich von 2 bis 50 bar, vorzugsweise im Bereich von 6 bis 30 bar, besonders bevorzugt im Bereich von 10 bis 25 bar.

Die Selektivhydrierung in Schritt g) kann einstufig, das heißt unter Verwendung eines einzigen Reaktors betrieben werden. Bevorzugt wird die Selektivhydrierung mehrstufig, also unter Verwendung von mindestens zwei parallel oder in Reihe geschalteten Reaktoren, betrieben. Die Reaktoren liegen dabei vorzugsweise in Reihe geschaltet vor. Dabei wird der Wasserstoff in jeden der in Reihe geschalteten Reaktoren in der zweiten Reaktionszone zusammen mit dem Dehydriergemisch eingespeist, während das Kohlenmonoxid nur in den ersten der in Reihe geschalteten Reaktoren zugegeben wird, nicht jedoch in den zweiten oder nachfolgenden Reaktoren.

Die Selektivhydrierung wird vorzugsweise außerdem als Flüssigphasenverfahren betrieben, das heißt, dass alle Komponenten in dem einen oder mehreren Reaktoren in flüssiger Phase vorliegen oder flüssig in die erste Reaktionszone eingebracht werden. Geeignete Reaktoren für die Durchführung der Selektivhydrierung sind Festbettreaktoren oder Rohrbündelreaktoren. Der Wasserstoff liegt ebenso wie das Kohlenmonoxid in der flüssigen Phase vorzugsweise vollständig gelöst vor. Es ist klar, dass die Temperatur und der Druck dann so gewählt werden müssen, dass der Wasserstoff und das Kohlenmonoxid vollständig gelöst bleiben und sich vor und in der Reaktionszone keine Gasphase ausbildet.

Erst nach Hydrierung des Butadiens wird das so erhaltene Hydriergemisch in Schritt h) zu einer MTBE- oder ETBE-Synthese geleitet, bei der Isobuten mit Methanol zu MTBE oder mit Ethanol zu ETBE umgesetzt wird und dann vom restlichen Hydriergemisch abgetrennt werden kann. Die Abtrennung erfolgt vorzugsweise destillativ. Die MTBE- oder ETBE-Synthese kann wie oben beschrieben durchgeführt werden.

Die im Hydriergemisch verbliebenen n-Butene werden dann in Schritt i) einer Oligomerisierung unterworfen, bei die n-Butene katalytisch zu C8-Olefinen oligomerisiert werden. Die Oligomerisierung erfolgt üblicherweise in mindestens einem geeigneten Reaktor. Es sind natürlich auch Ausführungsformen denkbar, bei der mehrere gleichzeitig betriebene Reaktoren parallel geschaltet oder in Reihe geschaltet vorliegen. Als Reaktor können alle dem Fachmann bekannten Reaktoren eingesetzt werden, die für die Oligomerisierung geeignet sind, bspw. Rohrreaktoren, Rohrbündelreaktoren, Riser-Settler-Reaktoren, Slurry-Reaktoren.

Der Reaktor enthält einen Oligomerisierungskatalysator zur Durchführung der Oligomerisierung, insbesondere einen heterogenen Oligomerisierunsgkatalysator. Der Oligomerisierungskatalysator liegt dabei insbesondere in Form eines Granulats, eines Extrudats oder in Tablettenform vor.

Der (heterogenen) Oligomerisierungskatalysator ist vorzugsweise ein Übergangsmetall-haltiger Oligomerisierungskatalysatoren. Das Übergangsmetall bzw. die entsprechend eingesetzten Übergangsmetallverbindung ist dabei vorzugsweise auf einem Trägermaterial, besonders bevorzugt einem auf Alumosilicat basierenden Trägermaterial, angeordnet. Als Übergangsmetallverbindungen für die erfindungsgemäße eingesetzten Oligomerisierungskatalysatoren eignen sich insbesondere Verbindungen von Nickel, Cobalt, Chrom, Titan und Tantal. Bevorzugt sind Nickel- und Cobalt-Verbindungen, besonders bevorzugt sind Nickel-Verbindungen. In einer bevorzugten Ausführungsform umfasst der erfindungsgemäße Oligomerisierungskatalysator eine Nickelverbindung, vorzugsweise Nickeloxid, auf einem Alumosilicat-Trägermaterial.

Die Oligomerisierung kann bei einer Temperatur im Bereich von 50 bis 200 °C, vorzugsweise 60 bis 180 °C, bevorzugt im Bereich von 60 bis 130°C durchgeführt werden. Der Druck kann von 10 bis 70 bar, bevorzugt von 20 bis 55 bar betragen.

Nach der Oligomerisierung sollte das erhaltene Oligomerisat dadurch aufgearbeitet werden, dass die gebildeten C8-Olefine von den restlichen im Oligomerisat befindlichen Stoffen (z. B. nicht umgesetzte Butene oder Alkane) abgetrennt werden. Das kann beispielsweise mittels Destillation erfolgen. Das restliche Oligomerisat, von dem die C8-Olefine abgetrennt worden sind, kann zur Oligomerisierung zurückgeführt werden. Die C8-Olefine werden im nächsten Schritt einer Hydroformylierung zugeführt. In einer bevorzugten Ausführungsform kann zusätzlich zu der Abtrennung der C8-Olefine eine Abtrennung von n-Butanen aus dem Oligomerisat, also vor der Hydroformylierung, erfolgen. Dadurch kann verhindert werden, dass sich n-Butane in der Oligomerisierung anreichern.

Die bei der Oligomerisierung gebildeten C8-Olefine werden dann in Schritt j) einer Hydroformylierung zugeführt, bei der C8-Olefine mit Synthesegas katalytisch zu C9-Aldehyden umgesetzt werden. Synthesegas bei der Hydroformylierung betrifft bekanntermaßen eine Mischung aus CO und H₂.

Die Hydroformylierung ist vorzugsweise eine homogen katalysierte Hydroformylierung, bei der das Katalysatorsystem (vollständig) gelöst in der flüssigen Phase des Reaktionsgemisches vorliegt. Das Katalysatorsystem der ersten Hydroformylierung umfasst vorzugsweise ein Übergangsmetall aus der 8. oder 9. Gruppe des Periodensystems der Elemente (PSE) und mindestens einen organischen Phosphor-haltigen Liganden.

Als Übergangsmetall können insbesondere Eisen, Ruthenium, Iridium, Cobalt oder Rhodium, vorzugsweise Cobalt oder Rhodium eingesetzt werden. Als katalytische aktive Spezies werden üblicherweise (Liganden)-Carbonyl-Komplexe der Metallatome diskutiert, die sich unter den bei der Hydroformylierung vorherrschenden Drücken und Temperaturen im flüssigen Reaktionsgemisch bilden.

Die Hydroformylierung kann in Anwesenheit eines Lösemittels durchgeführt werden, wobei das Lösemittel mit dem Hydroformylierungsverfahren kompatibel sein sollte. Als Lösemittel können die dem Fachmann bekannten Lösemittel für die klassische Hydroformylierung verwendet werden, beispielsweise Alkane, aromatische Kohlenwasserstoffe, Wasser, Ether, Ester, Ketone, Alkohole und die Reaktions- oder Nebenprodukte der Hydroformylierung wie Aldehyde und Kondensationsprodukte der Aldehyde.

Die Hydroformylierung kann bei einem Druck von 10 bis 400 bar, vorzugsweise 15 bis 250 bar durchgeführt werden. Die Temperatur bei der Hydroformylierung kann 70 bis 250 °C, vorzugsweise 100 bis 200 °C betragen.

Die Hydroformylierung sollte ebenfalls in mindestens einem Reaktor durchgeführt werden. Es sind natürlich auch Ausführungsformen denkbar, bei der mehrere gleichzeitig betriebene Reaktoren parallel geschaltet oder in Reihe geschaltet vorliegen. Als Reaktor für die Hydroformylierung kommen alle dem Fachmann bekannten und für die Hydroformylierung geeigneten Reaktortypen, insbesondere Gas-Flüssig-Reaktoren, in Betracht. Geeignete Reaktortypen sind insbesondere Rührkesselreaktoren, Blasensäulenreaktoren, kaskadierte Blasensäulenreaktoren, Jetloopreaktoren oder Schlaufenreaktoren.

Die bei der Hydroformylierung in Schritt j) gebildeten C9-Aldehyde können danach in bekannter Weise aufgearbeitet und vom Reaktionsgemisch abgetrennt werden. Die erhaltenen C9-Aldehyde werden nachfolgend in Schritt k) einer Hydrierung zugeführt, bei der die C9-Aldehyde katalytisch unter Zugabe von Wasserstoff zu Isononanol umgesetzt werden.

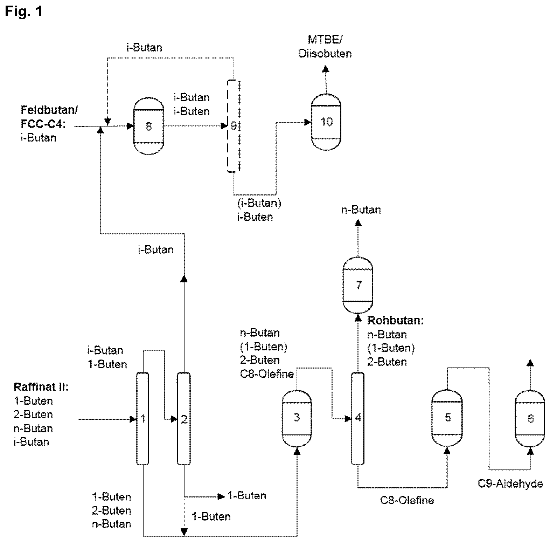

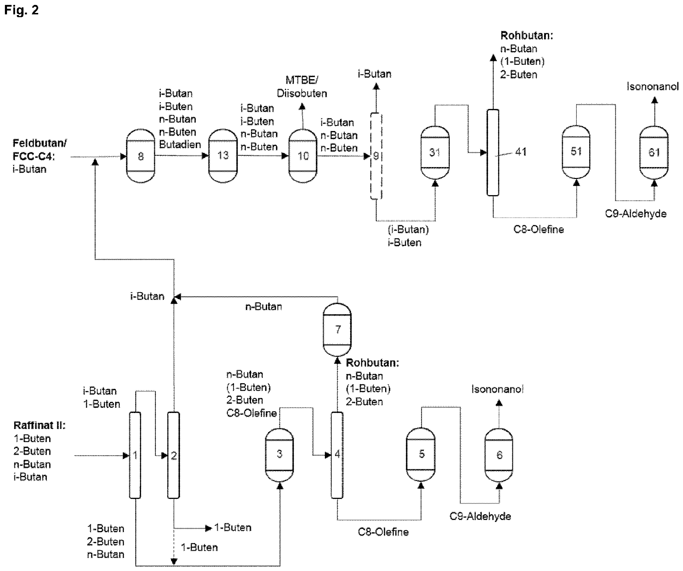

Exemplarische Ausführungsformen des erfindungsgemäßen Verfahrens sind den Fig.1 und 2 als Blockschema gezeigt. Es versteht sich, dass diese Ausführungsformen nur der Erläuterung dienen und nicht einschränkend zu verstehen sind.

Das Blockschema in Figur 1 zeigt eine Ausführungsform bei der ein Raffinat II, welches zumindest 1-Buten, 2-Buten, n-Butan und Isobutan enthält, zunächst einer ersten Destillation (1) zugeführt wird, bei der zumindest ein Teil des 1-Butens und zumindest ein Teil des Isobutans am Kopf abgenommen werden. In einer zweiten Destillation (2) werden Isobutan und 1-Buten voneinander getrennt. Das Isobutan wird zum zweiten Verfahrensstrang geführt. Das 1-Buten fällt bei der Destillation (2) am Sumpf an und kann dort entweder entnommen werden und/oder zusammen mit dem Sumpfprodukt der ersten Destillation (1) zur Oligomerisierung (3) geführt werden.

Bei der Oligomerisierung (3) entsteht ein Oligomerisat, das n-Butan, nicht umgesetzte Butene (1-Buten und 2-Buten) und die gebildeten Oligomere (hauptsächlich C8-, aber auch C12-, C16- und höhere Olefine) enthält und zur Produktauftrennung (4) geführt. Zur Vereinfachung des Blockschemas wurde der genaue Ablauf der Produktauftrennung in die verschiedenen Oligomerenfraktionen verzichtet. Durch die Produktabtrennung entsteht Rohbutan, das neben n-Butan auch Butene enthält. Das Rohbutan geht üblicherweise über Kopf und wird zur Vollhydrierung (7) gefahren und dort zu n-Butan hydriert.

Die vom Oligomerisat abgetrennten C8-Olefine werden zur Hydroformylierung (5) geleitet und gehen nach einer Produktauftrennung (nicht eingezeichnet) zur Hydrierung (6), wo aus den Aldehyden Isononanol gebildet wird.

Das vom Raffinat II abgetrennte Isobutan wird zusammen mit Isobutan aus Feldbutanen oder FCC-C4-Strömen zur Dehydrierung (8) geleitet, wodurch Isobuten entsteht. Von dem Dehydriergemisch aus der Dehydrierung (8) kann in einer optionalen Destillation (9) Isobutan abgetrennt und zur Dehydrierung (8) zurückgeführt werden. Die optionale Ausgestaltung ist durch die gestrichelten Linien angedeutet. Das Isobuten wird in der nachfolgenden Reaktionszone (10) entweder mit Methanol zur MTBE umgesetzt oder dimerisiert. Sollte das Isobutan vorher nicht abgetrennt worden sein, würde die Abtrennung nach der Reaktionszone (10) durchgeführt und Isobutan zur Dehydrierung (8) zurückgeführt (nicht eingezeichnet).

Das Blockschema in Figur 2 zeigt eine bevorzugte Ausführungsform der vorliegenden Erfindung. Der untere Verfahrensstrang mit Isobutanabtrennung (1,2), Oligomerisierung (3), Rohbutan- bzw. Produktabtrennung (4), Hydroformylierung (5) und Hydrierung (6) entspricht dem für Figur 1 beschriebenen Verfahren. Der Unterschied besteht hier darin, dass das abgetrennte Rohbutan nach der Vollhydrierung (7) zusammen mit dem abgetrennten Isobutan und Isobutan aus Feldbutanen oder FCC-C4-Strömen zur Dehydrierung (8) geleitet, wodurch neben Isobuten auch n-Butene (1-Buten und 2-Buten) und Butadien gebildet wird.

Das Butadien wird in der Selektivhydrierung (13) dann zunächst zu Butenen und in geringen Mengen zu Butan hydriert. Der daraus erhaltene Strom wird dann einer Reaktionszone (10) zugeführt, wo Isobuten entweder mit Methanol zur MTBE oder mit Ethanol zu ETBE umgesetzt wird. Nach der Abtrennung von MTBE bzw. ETBE wird optional Isobutan mittels Destillation (9) aus dem Strom entfernt. Das Isobutan kann zur Dehydrierung (8) zurückgeführt werden (nicht eingezeichnet). Der restliche Strom wird dann zur Oligomerisierung (31) geleitet und das Oligomerisat in der Produktabtrennung (41) aufgetrennt und von Rohbutan befreit. Das Rohbutan könnte über eine Vollhydrierung zur Dehydrierung geführt werden (nicht eingezeichnet).

Die in der Oligomerisierung (31) gebildeten C8-Olefine werden nachfolgende einer Hydroformylierung (51) unterworfen und die gebildeten C9-Aldehyde zur Hydrierung (61) geleitet, um Isononanol zu bilden.

## Patentansprüche

1. Verfahren zur flexiblen Herstellung von MTBE und Isononanol, welches die folgenden Schritte umfasst:
a) Bereitstellen eines C4-Kohlenwasserstoffstroms, welcher zumindest n-Butene (1-Buten und 2-Buten), n-Butan und Isobutan enthält, als ersten Einsatzstrom und destillative Abtrennung zumindest eines Teils des Isobutans und zumindest eines Teils des 1-Butens aus dem ersten Einsatzstrom, wobei der C4-Kohlenwasserstoffstrom ein Raffinat-II-Strom ist.;
b) Verwendung des aus dem ersten Einsatzstrom abgetrennten Isobutans und zusätzliches Isobutan, welches aus einem Feldbutanstrom oder einem FCC-C4-Strom abgetrennt worden ist, als zweiten Einsatzstrom;
c) Zuführen des ersten Einsatzstroms zu einer Oligomerisierung, in der die n-Butene katalytisch zu C8-Olefinen oligomerisiert werden;
d) Zuführen der in der Oligomerisierung in Schritt c) gebildeten C8-Olefine zu einer Hydroformylierung, in der C8-Olefine mit Synthesegas katalytisch zu C9-Aldehyden umgesetzt werden;
e) Zuführen der C9-Aldehyde aus der Hydroformylierung in Schritt d) zu einer Hydrierung, bei der C9-Aldehyde katalytisch zu Isonanol umgesetzt werden;
f) Zuführen des zweiten Einsatzstroms zu einer Dehydrierung, wodurch ein Dehydriergemisch erhalten wird, welches zumindest Isobuten und nicht umgesetztes Isobutan enthält und optional Abtrennen des Isobutans vom Dehydriergemisch;
g) Zuführen des Isobutens aus Schritt f) zu einer MTBE- oder ETBE-Synthese, in der Isobuten mit Methanol katalytisch zu MTBE oder mit Ethanol katalytisch zu ETBE umgesetzt wird, oder zu einer Isobutendimerisierung, bei der Isobuten katalytisch dimerisiert wird.

2. Verfahren nach Anspruch 1, wobei der C4-Kohlenwasserstoffstrom in Schritt a) ein Raffinat-II-Strom aus einer Crack-C4-Fraktion eines Steamcrackers ist.

3. Verfahren nach Anspruch 1 oder 2, wobei der bei der Oligomerisierung in Schritt c) eingesetzte Oligomerisierungskatalysator ein heterogener Übergangsmetall-haltiger Oligomerisierungskatalysator ist, wobei das Übergangsmetall aus Nickel, Cobalt, Chrom, Titan oder Tantal ist, ausgewählt wird

4. Verfahren nach einem der Ansprüche 1 bis 3, wobei die Oligomerisierung in Schritt c) bei einer Temperatur im Bereich von 50 bis 200 °C und bei einem Druck von 10 bis 70 bar durchgeführt wird.

5. Verfahren nach einem der Ansprüche 1 bis 4, wobei die Hydroformylierung in Schritt d) eine homogene Hydroformylierung ist, bei der das erste Katalysatorsystem (vollständig) gelöst in der flüssigen Phase des Reaktionsgemisches vorliegt.

6. Verfahren nach Anspruch 5, wobei das bei der Hydroformylierung in Schritt d) eingesetzte Katalysatorsystem ein Übergangsmetall aus der 8. oder 9. Gruppe des Periodensystems der Elemente (PSE) und mindestens einen organischen Phosphor-haltigen Liganden umfasst.

7. Verfahren nach Anspruch 6, wobei als Übergangsmetall Eisen, Ruthenium, Iridium, Cobalt oder Rhodium eingesetzt werden.

8. Verfahren nach einem der Ansprüche 1 bis 7, wobei das bei der Aufarbeitung des in Schritt c) erhaltenen Oligomerisats anfallende restliche Gemisch (Rohbutan), welches zumindest n-Butan und nicht umgesetzte Butene enthält, einer Hydrierung unterworfen wird.

9. Verfahren nach Anspruch 8, wobei die n-Butane aus dem hydrierten Rohbutan zusammen mit dem aus dem ersten Einsatzstrom abgetrennten Isobutan und zusätzlichem Isobutan, welches aus einem Feldbutanstrom oder einem FCC-C4-Strom abgetrennt worden ist, als zweiter Einsatzstrom eingesetzt wird; wobei die Schritte f) und g) nach Anspruch 1 durch die folgenden Verfahrensschritte ersetzt werden:
f) Zuführen des zweiten Einsatzstroms zu einer Dehydrierung, wodurch ein Dehydriergemisch erhalten wird, welches zumindest n-Butene (1-Buten und 2-Buten) Isobuten, Butadien, nicht umgesetztes Butan und nicht umgesetztes Isobutan enthält und optional Abtrennen des nicht umgesetzten n-Butans und/oder Isobutans vom Dehydriergemisch;
g) Zuführen des Dehydriergemisches aus Schritt f) zu einer selektiven Hydrierung, in der Butadien selektiv zu Buten hydriert wird, unter Erhalt eines Hydriergemisches;
h) Zuführen des Hydriergemisches aus Schritt g) zu einer MTBE- oder ETBE-Synthese, in der Isobuten mit Methanol katalytisch zu MTBE oder mit Ethanol katalytisch zu ETBE umgesetzt wird und Abtrennen des gebildeten MTBE oder ETBE vom restlichen Hydriergemisch;
i) Zuführen des restlichen Hydriergemisches zu einer Oligomerisierung, in der die n-Butene katalytisch zu C8-Olefinen oligomerisiert werden;
j) Zuführen der in der Oligomerisierung in Schritt i) gebildeten C8-Olefine zu einer Hydroformylierung, in der C8-Olefine mit Synthesegas katalytisch zu C9-Aldehyden umgesetzt werden;
k) Zuführen der C9-Aldehyde aus der Hydroformylierung in Schritt j) zu einer Hydrierung, bei der C9-Aldehyde katalytisch zu Isonanol umgesetzt werden.

10. Verfahren nach Anspruch 9, wobei nach der Oligomerisierung in Schritt i) und vor der Hydroformylierung in Schritt j) eine Abtrennung von n-Butanen durchgeführt wird.

## Claims

1. Process for flexible production of MTBE and isononanol comprising the steps of:
a) providing a C4-hydrocarbon stream containing at least n-butenes (1-butene and 2-butene), n-butane and isobutane as a first input stream and distillatively separating at least a portion of the isobutane and at least a portion of the 1-butene from the first input stream, wherein the C4-hydrocarbon stream is a raffinate-II stream;
b) using the isobutane separated from the first input stream and additional isobutane separated from a field butane stream or an FCC-C4 stream as a second input stream;
c) sending the first input stream to an oligomerization in which the n-butenes are catalytically oligomerized to afford C8-olefins;
d) sending the C8-olefins formed in the oligomerization in step c) to a hydroformylation in which C8-olefins are catalytically reacted with synthesis gas to afford C9-aldehydes;
e) sending the C9-aldehydes from the hydroformylation in step d) to a hydrogenation in which C9-aldehydes are catalytically converted into isononanol;
f) sending the second input stream to a dehydrogenation to obtain a dehydrogenation mixture containing at least isobutene and unconverted isobutane and optionally separating the isobutane from the dehydrogenation mixture;
g) sending the isobutene from step f) to an MTBE or ETBE synthesis in which isobutene is catalytically reacted with methanol to afford MTBE or with ethanol to afford ETBE or to an isobutene dimerization in which isobutene is catalytically dimerized.

2. Process according to Claim 1, wherein the C4-hydrocarbon stream in step a) is a raffinate-II stream from a cracked C4-fraction from a steamcracker.

3. Process according to Claim 1 or 2, wherein the oligomerization catalyst employed in the oligomerization in step c) is a heterogeneous transition metal-containing oligomerization catalyst, wherein the transition metal is selected from nickel, cobalt, chromium, titanium or tantalum.

4. Process according to any of Claims 1 to 3, wherein the oligomerization in step c) is performed at a temperature in the range from 50°C to 200°C and at a pressure of 10 to 70 bar.

5. Process according to any of Claims 1 to 4, wherein the hydroformylation in step d) is a homogeneous hydroformylation in which the first catalyst system is (completely) dissolved in the liquid phase of the reaction mixture.

6. Process according to Claim 5, wherein the catalyst system employed in the hydroformylation in step d) comprises a transition metal from the 8th or 9th group of the periodic table of the elements (PTE) and at least one organic phosphorus-containing ligand.

7. Process according to Claim 6, wherein the employed transition metal is iron, ruthenium, iridium, cobalt or rhodium.

8. Process according to any of Claims 1 to 7, wherein the remaining mixture (raw butane) generated during workup of the oligomer obtained in step c) and containing at least n-butane and unconverted butenes is subjected to a hydrogenation.

9. Process according to Claim 8, wherein the n-butanes from the hydrogenated raw butane together with the isobutane separated from the first input gas stream and additional isobutane separated from a field butane stream or an FCC-C4 stream are employed as the second input stream; wherein steps f) and g) according to Claim 1 are replaced by the following process steps:
f) sending the second input stream to a dehydrogenation to obtain a dehydrogenation mixture containing at least n-butenes (1-butene and 2-butene), isobutene, butadiene, unconverted butane and unconverted isobutane and optionally separating the unconverted n-butane and/or isobutane from the dehydrogenation mixture;
g) sending the dehydrogenation mixture from step f) to a selective hydrogenation in which butadiene is selectively hydrogenated to afford butene to obtain a hydrogenation mixture;
h) sending the hydrogenation mixture from step g) to an MTBE or ETBE synthesis in which isobutene is catalytically reacted with methanol to afford MTBE or with ethanol to afford ETBE and separating the resulting MTBE or ETBE from the remaining hydrogenation mixture;
i) sending the remaining hydrogenation mixture to an oligomerization in which the n-butenes are catalytically oligomerized to afford C8-olefins;
j) sending the C8-olefins formed in the oligomerization in step i) to a hydroformylation in which C8-olefins are catalytically reacted with synthesis gas to afford C9-aldehydes;
k) sending the C9-aldehydes from the hydroformylation in step j) to a hydrogenation in which C9-aldehydes are catalytically converted into isononanol.

10. Process according to Claim 9, wherein after the oligomerization in step i) and before the hydroformylation in step j) a separation of n-butanes is performed.

## Revendications

1. Procédé pour la préparation flexible de MTBE et d'isononanol, qui comprend les étapes suivantes :
a) préparation d'un flux hydrocarboné en C4 qui contient au moins des n-butènes (1-butène et 2-butène), du n-butane et de l'isobutane, comme premier flux d'alimentation et séparation par distillation d'au moins une partie de l'isobutane et d'au moins une partie du 1-butène du premier flux d'alimentation, le flux hydrocarboné en C4 étant un flux de raffinat II ;
b) utilisation de l'isobutane séparé du premier flux d'alimentation et d'isobutane supplémentaire, qui a été séparé d'un flux de butane naturel ou d'un flux en C4 de FCC (craquage catalytique fluide), comme deuxième flux d'alimentation ;
c) introduction du premier flux d'alimentation dans une oligomérisation, dans laquelle les n-butènes sont oligomérisés catalytiquement en oléfines en C8 ;
d) introduction des oléfines en C8 formées dans l'oligomérisation dans l'étape c) dans une hydroformylation dans laquelle les oléfines en C8 sont transformées catalytiquement avec du gaz de synthèse en aldéhydes en C9 ;
e) introduction des aldéhydes en C9 provenant de l'hydroformylation dans l'étape d) dans une hydrogénation dans laquelle les aldéhydes en C9 sont transformés catalytiquement en isononanol ;
f) introduction du deuxième flux d'alimentation dans une déshydrogénation suite à quoi on obtient un mélange de déshydrogénation qui contient au moins de l'isobutène et de l'isobutane non transformé et, éventuellement, séparation de l'isobutane du mélange de déshydrogénation ;
g) introduction de l'isobutène de l'étape f) dans une synthèse de MTBE ou d'ETBE, dans laquelle l'isobutène est transformé catalytiquement avec du méthanol en MTBE ou avec de l'éthanol en ETBE ou dans une dimérisation d'isobutène dans laquelle l'isobutène est dimérisé catalytiquement.

2. Procédé selon la revendication 1, le flux hydrocarboné en C4 dans l'étape a) étant un flux de raffinat II provenant d'une fraction de craquage en C4 d'un dispositif de craquage à la vapeur.

3. Procédé selon la revendication 1 ou 2, le catalyseur d'oligomérisation utilisé lors de l'oligomérisation dans l'étape c) étant un catalyseur d'oligomérisation hétérogène contenant un métal de transition, le métal de transition étant choisi parmi le nickel, le cobalt, le chrome, le titane ou le tantale.

4. Procédé selon l'une quelconque des revendications 1 à 3, l'oligomérisation dans l'étape c) étant réalisée à une température dans la plage de 50 à 200°C et à une pression de 10 à 70 bars.

5. Procédé selon l'une quelconque des revendications 1 à 4, l'hydroformylation dans l'étape d) étant une hydroformylation homogène, dans laquelle le premier système catalytique se trouve sous forme (complètement) dissoute dans la phase liquide du mélange réactionnel.

6. Procédé selon la revendication 5, le système catalytique utilisé lors de l'hydroformylation dans l'étape d) comprenant un métal de transition du 8ème ou du 9ème groupe du système périodique des éléments (SPE) et comprenant au moins un ligand organique contenant du phosphore.

7. Procédé selon la revendication 6, le fer, le ruthénium, l'iridium, le cobalt ou le rhodium étant utilisés comme métal de transition.

8. Procédé selon l'une quelconque des revendications 1 à 7, le mélange résiduel (butane brut) qui contient au moins du n-butane et des butènes non transformés, obtenu lors du traitement de l'oligomère obtenu dans l'étape c) étant soumis à une hydrogénation.

9. Procédé selon la revendication 8, les n-butanes du butane brut hydrogéné étant utilisés conjointement avec l'isobutane séparé du premier flux d'alimentation et de l'isobutane supplémentaire, qui a été séparé d'un flux de butane naturel ou d'un flux en C4 de FCC (craquage catalytique fluide), comme deuxième flux d'alimentation ; les étapes f) et g) selon la revendication 1 étant remplacées par les étapes de procédé suivantes :
f) introduction du deuxième flux d'alimentation dans une déshydrogénation suite à quoi on obtient un mélange de déshydrogénation qui contient au moins des n-butènes (1-butène et 2-butène), de l'isobutène, du butadiène, du butane non transformé et de l'isobutane non transformé et, éventuellement, séparation du n-butane et/ou de l'isobutane non transformé(s) du mélange de déshydrogénation ;
g) introduction du mélange de déshydrogénation de l'étape f) dans une hydrogénation sélective, dans laquelle le butadiène est hydrogéné sélectivement en butène, avec obtention d'un mélange d'hydrogénation ;
h) introduction du mélange d'hydrogénation de l'étape g) dans une synthèse de MTBE ou d'ETBE dans laquelle l'isobutène est transformé catalytiquement avec du méthanol en MTBE ou avec de l'éthanol en ETBE et séparation du MTBE ou de l'ETBE du mélange d'hydrogénation résiduel ;
i) introduction du mélange d'hydrogénation résiduel dans une oligomérisation, dans laquelle les n-butènes sont oligomérisés catalytiquement en oléfines en C8 ;
j) introduction des oléfines en C8 formées dans l'oligomérisation dans l'étape i) dans une hydroformylation dans laquelle les oléfines en C8 sont transformées catalytiquement avec du gaz de synthèse en aldéhydes en C9 ;
k) introduction des aldéhydes en C9 provenant de l'hydroformylation dans l'étape j) dans une hydrogénation dans laquelle les aldéhydes en C9 sont transformés catalytiquement en isononanol.

10. Procédé selon la revendication 9, une séparation de n-butanes étant réalisée après l'oligomérisation dans l'étape i) et avant l'hydroformylation dans l'étape j).
